# EUROPÄISCHE PATENTSCHRIFT

(11) **EP 1 361 856 B1**
(45) Veröffentlichungstag und Bekanntmachung des Hinweises auf die Patenterteilung: **15.08.2007**
(21) Anmeldenummer: 02719812.6
(22) Anmeldetag: 13.02.2002
(51) Int. Cl.: A61K 8/73, A61K 8/81, A61Q 19/00, A61K 9/107

(54) **GELEMULSIONEN IN FORM VON O/W-EMULSIONEN MIT EINEM GEHALT AN HYDROKOLLOIDEN**
GEL EMULSIONS IN THE FORM OF O/W EMULSIONS HAVING A HYDROCOLLOID CONTENT
EMULSIONS EN GEL SE PRESENTANT SOUS LA FORME D'EMULSIONS HUILE DANS L'EAU CONTENANT DES HYDROCOLLOIDES

(30) Priorität: 16.02.2001 DE 10107240
(43) Veröffentlichungstag der Anmeldung: 19.11.2003
(73) Patentinhaber: Beiersdorf AG, 20253 Hamburg (DE)
(72) Erfinder: FÜLLER, Silke, 22523 Hamburg (DE); VON THADEN, Stefanie, 20255 Hamburg (DE); BLECKMANN, Andreas, 22926 Ahrensburg (DE); LEMM, Christel, 21614 Buxtehude (DE); EMEIS, Detlef, 22335 Hamburg (DE)
(74) Vertreter: Wilke, Jochen
(86) Internationale Anmeldenummer: PCT/EP2002/001474
(87) Internationale Veröffentlichungsnummer: WO 2002/066007

(56) Entgegenhaltungen:
- EP-A- 0 747 035
- EP-A- 1 055 417
- EP-A- 1 077 062
- WO-A-94/17779
- DE-A- 19 802 204

## Beschreibung

Die vorliegende Erfindung betrifft kosmetische und dermatologische Zubereitungen, insbesondere solche vom Typ Öl-in-Wasser, Verfahren zu ihrer Herstellung sowie ihre Verwendung für kosmetische und medizinische Zwecke.

Die menschliche Haut übt als größtes Organ des Menschen zahlreiche lebenswichtige Funktionen aus. Mit durchschnittlich etwa 2 m² Oberfläche beim Erwachsenen kommt ihr eine herausragende Rolle als Schutz- und Sinnesorgan zu. Aufgabe dieses Organs ist es, mechanische, thermische, aktinische, chemische und biologische Reize zu vermitteln und abzuwehren. Außerdem kommt ihr eine bedeutende Rolle als Regulations- und Zielorgan im menschlichen Stoffwechsel zu.

Unter kosmetischer Hautpflege ist in erster Linie zu verstehen, die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) zu stärken oder wiederherzustellen sowie ihre Hornschicht bei aufgetretenen Schäden in ihrem natürlichen Regenerationsvermögen zu unterstützen.

Werden die Barriereeigenschaften der Haut gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Gelcremes sind besonders leichte Produkte mit einem niedrigen Emulgator- und Lipidgehalt. Sie zeichnen sich dadurch aus, daß sie sich leicht auf der Haut verteilen lassen und ein Frischegefühl vermitteln. Nach dem Produktauftrag soll auf der Haut kein oder nur wenig Rückstand verbleiben. Gelcremes enthalten in der Regel einen relativ hohen Anteil an hydrophilen Verdickungsmitteln (z.B. Carbopole, Xanthan Gum, Hydroxyethyl Cellulose) zur Verdickung und Stabilisierung der Systeme. Da sich der Verdicker oder das Verdickersystem in der äußeren Phase befindet, hat es einen signifikanten Einfluss auf die sensorischen Eigenschaften des Produktes. Gängige Verdickersysteme lassen sich entweder nicht leicht verteilen, ergeben kein Frischegefühl oder hinterlassen einen zu schmierigen Rückstand an den Fingern und/oder ein stumpfes, klebriges Hautgefühl nach dem Verteilen des Produktes auf der Haut.

Diesen Nachteilen galt es abzuhelfen.

DE- 198 02 204 und EP- 1 055 417 offenbaren gelförmige kosmetische O/W- Zubereitungen, die ein oder zwei Hydrokolloide enthalten.

Überraschend hat sich gezeigt, daß kosmetische Zubereitungen in Form von Gelemulsionen, nämlich auf Zubereitungen des Typs O/W-Emulsion mit einem Gehalt von
- Hydrokolloiden, gewählt aus der Gruppe
   (i) Hydroxyethylcellulose
   (ii) Xanthan Gummi
   (iii) Carbomer
   basierende Zubereitungen,
   - wobei das Verhältnis aus (i) : (ii) : (iii) wie a : b : c gewählt wird, wobei a, b und c unabhängig voneinander rationale Zahlen von 1 bis 5, bevorzugt von 1 bis 3 darstellen, und wobei b auch den Wert Null annehmen kann,
   - wobei (i), (ii) und (iii) in den Zubereitungen in Gesamtkonzentrationen (a + b + c) von 0,25 - 1,5 Gew.-% vorliegen, bezogen auf das Gesamtgewicht der Zubereitungen, bevorzugt in Gesamtkonzentrationen von 0,5 - 1,0 Gew.-%,
- wobei diese Zubereitungen ferner enthalten
   (iv) ein oder mehrere Lipide,
      - wobei der Gesamtgehalt der Lipide gewählt wird aus dem Konzentrationsbereich von 3,0 bis 20,0 Gew.-%, bezogen auf das Gesamtgewicht der Zubereitungen, bevorzugt in Gesamtkonzentrationen von 7,5 bis 15 Gew.-%.
- wobei diese Zubereitungen ferner enthalten
   (v) einen oder mehrere nichtionische und/oder anionische Emulgatoren mit HLB-Werten zwischen 8 und 16, bevorzugt zwischen 10 und 12
      - wobei der Gesamtgehalt der Emulgatoren 1,5 Gew.-% nicht übersteigt und bevorzugt gewählt wird aus dem Konzentrationsbereich von 0,5 bis 1,0 Gew.-% bezogen auf das Gesamtgewicht der Zubereitungen,
diese Aufgaben lösen.

Es war für den Fachmann nicht vorauszusehen gewesen, daß die erfindungsgemäßen Zubereitungen
- besser als feuchtigkeitsspendende Zubereitungen wirken,
- einfacher zu formulieren sein,
- besser die Hautglättung fördern,
- sich durch bessere Pflegewirkung auszeichnen,
- besser als Vehikel für kosmetische und medizinisch-dermatologische Wirkstoffe dienen
- bessere sensorische Eigenschaften, wie beispielsweise die Verteilbarkeit auf der Haut oder das Einzugsvermögen in die Haut, aufweisen würden
- höhere Stabilität gegenüber Zerfall in Öl- und Wasserphasen aufweisen und
- sich durch bessere Bioverträglichkeit auszeichnen würden
als die Zubereitungen des Standes der Technik.

Die erfindungsgemäßen Zubereitungen stellen daher eine Bereicherung des Standes der Technik dar.

Xanthan Gummi (CAS-Nr. 11138-66-2), auch Xanthan genannt, stellt ein anionisches Heteropolysaccharid dar, das in der Regel durch Fermentation aus Maiszucker gebildet und als Kaliumsalz isoliert wird. Es wird von Xanthomonas campestris und einigen anderen Spezies unter aeroben Bedingungen mit einem Molekulargewicht von 2×10⁶ bis 24x10⁶ produziert. Xanthan Gummi wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Xanthan Gummi ist die Bezeichnung für das erste mikrobielle anionische Heteropolysaccharid. Es wird von Xanthomonas campestris und einigen anderen Spezies unter aeroben Bedingungen mit einem Molekulargewicht von 2-15 10⁶ produziert. Xanthan Gummi wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Die Anzahl der Pyruvat-Einheiten bestimmt die Viskosität des Xanthan Gummis. Xanthan Gummi wird in zweitägigen Batch-Kulturen mit einer Ausbeute von 70-90 %, bezogen auf eingesetztes Kohlenhydrat, produziert. Dabei werden Ausbeuten von 25-30 g/l erreicht. Die Aufarbeitung erfolgt nach Abtöten der Kultur durch Fällung mit z. B. 2-Propanol. Xanthan Gummi wird anschließend getrocknet und gemahlen.

Im Rahmen der vorliegenden Offenbarung wird als Oberbegriff für Fette, Öle, Wachse und dergleichen gelegentlich der Ausdruck "Lipide" verwendet, wie dem Fachmanne durchaus geläufig ist. Auch werden die Begriffe "Ölphase" und "Lipidphase" synonym angewandt.

Vorteilhaft werden das oder die Lipide gewählt aus der Gruppe der mittelpolaren bis unpolaren Lipide. Es ist bevorzugt, den Gewichtsanteil polarer Lipide an der Lipidphase geringer als ca. 30 % auszugestalten.

Öle und Fette unterscheiden sich unter anderem in ihrer Polarität, welche schwierig zu definieren ist. Es wurde bereits vorgeschlagen, die Grenzflächenspannung gegenüber Wasser als Maß für den Polaritätsindex eines Öls bzw. einer Ölphase anzunehmen. Dabei gilt, daß die Polarität der betreffenden Ölphase umso größer ist, je niedriger die Grenzflächenspannung zwischen dieser Ölphase und Wasser ist. Erfindungsgemäß wird die Grenzflächenspannung als ein mögliches Maß für die Polarität einer gegebenen Ölkomponente angesehen.

Die Grenzflächenspannung ist diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m (Millinewton geteilt durch Meter) wiedergegeben. Sie hat positives Vorzeichen, wenn sie das Bestreben hat, die Grenzfläche zu verkleinern. Im umgekehrten Falle hat sie negatives Vorzeichen.

Die nachfolgende Tabelle 1 führt mittelpolare Lipide auf, die als Einzelsubstanzen oder auch im Gemisch untereinander erfindungsgemäß vorteilhaft sind. Die betreffenden Grenzflächenspannungen gegen Wasser sind in der letzten Spalte angegeben. Es ist gegebenenfalls auch vorteilhaft, Gemische aus höher- und niederpolaren und dergleichen zu verwenden, insbesondere sofern die Gesamtpolarität der Ölphase der einer mittleren oder niedrigen Polarität entspricht.

| **Tabelle 1** | | |
|---|---|---|
| **Handelsname** | **INCI-Bezeichnung** | **(mN/m)** |
| Isofol® 14 T | Butyl Decanol + Hexyl Decanol + Hexyl Octanol + Butyl Octanol | 27,6 |
| Isofol® 16 | Hexyl Decanol | 24,3 |
| Eutanol® G | Octyldodecanol | 24,8 |
| Cetiol® OE | Dicaprylyl Ether | 22,1 |
| Miglyol® 812 | Caprylic/Capric Triglyceride | 21,3 |
| Cegesoft® C24 | Octyl Palmitate | 23,1 |
| Isopropylstearat | Isopropyl Stearate | 21,9 |
| Estol® 1540 EHC | OctylOctanoate | 30,0 |
| Finsolv® TN | C₁₂₋₁₅ Alkyl Benzoate | 21,8 |
| Cetiol® SN | Cetearyl Isonoanoate | 28,6 |
| Dermofeel® BGC | Butylene Glycol Caprylate/Caprate | 21,5 |
| Trivent® OCG | Tricaprylin | 20,2 |
| MOD | Octyldodeceyl Myristate | 22,1 |
| Cosmacol® ETI | Di-C₁₂₋₁₃Alkyl Tartrate | 29,4 |
| Miglyol® 829 | Caprylic/Capric Diglyceryl Succinate | 29,5 |
| Prisorine® 2036 | Octyl Isostearate | 29,7 |
| Tegosoft® SH | Stearyl Heptanoate | 28,7 |
| Abil® Wax 9840 | Cetyl Dimethicone | 25,1 |
| Cetiol® LC | Coco-Caprylate/Caprate | 24,8 |
| IPP | Isopropyl Palmitate | 22,5 |
| Luvitol® EHO | Cetearyl Octanoate | 28,6 |
| Cetiol® 868 | Octyl Stearate | 28,4 |

Die Ölphase kann im Sinne der vorliegenden Erfindung ferner vorteilhaft Substanzen enthalten, gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, E-rucyloleat, Erucylerucat sowie synthetische, halbsynthetische und natürliche Gemische solcher Ester, wie z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr.

Gewünschtenfalls können in der Ölphase einzusetzende Fett- und/oder Wachskomponenten - als Nebenbestandteile in geringerer Menge - aus der Gruppe der pflanzlichen Wachse, tierischen Wachse, Mineralwachse und petrochemischen Wachse gewählt werden. Erfindungsgemäß günstig sind beispielsweise Candelillawachs, Carnaubawachs, Japanwachs, Espartograswachs, Korkwachs, Guarumawachs, Reiskeimölwachs, Zucker rohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse.

Weitere vorteilhafte Fett- und/oder Wachskomponenten sind chemisch modifizierte Wachse und synthetische Wachse, wie beispielsweise die unter den Handelsbezeichnungen Syncrowax HRC (Glyceryltribehenat), Syncrowax HGLC (C₁₆₋₃₆ -Fettsäuretriglycerid) und Syncrowax AW 1C (C₁₈₋₃₆ -Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder C₃₀₋₅₀ -Alkyl Bienenwachs), Polyalkylenwachse, Polyethylenglykolwachse, aber auch chemisch modifizierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie z.B. C₂₀₋₄₀-Alkylstearat, C₂₀₋₄₀-Alkylhydroxystearoylstearat und/oder Glycolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan.

Gewünschtenfalls können die Fett- und/oder Wachskomponenten sowohl einzeln als auch im Gemisch vorliegen.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als Lipidkomponente der Ölphase einzusetzen.

Von den Kohlenwasserstoffen sind Paraffinöl, hydrierte Polyolefine (z.B. hydriertes Polyisobuten) Squalan und Squalen vorteilhaft im Sinne der vorliegenden Erfindung zu verwenden.

Erfindungsgemäß besonders vorteilhaft sind solche Emulsionen, die dadurch gekennzeichnet sind, daß die Ölphase zu mindestens 50 Gew.-%, bevorzugt zu mehr als 75 Gew.-% aus mindestens einer Substanz, gewählt aus der Gruppe gewählt aus der Gruppe Vaseline (Petrolatum), Paraffinöl und Polyolefine, unter den letzteren bevorzugt: Polydecenen, besteht.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft kann Cyclomethicon (z.B. Octamethylcyclotetrasiloxan,Cyclopentasiloxan sowie Cyclohexasiloxan) eingesetzt werden. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan:
Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate.

Erfindungsgemäß vorteilhaft können das oder die Carbopole beispielsweise gewählt werden aus den Carbopoltypen der Firma Goodrich (Carbopol 980, 981, 1382, 5984, 2984, EDT 2001 oder Pemulen TR2).

Der oder die anionischen bzw. nichtionischen Emulgatoren können vorteilhaft gewählt werden aus der Gruppe der
a) Partialfettsäureester und Fettsäureester mehrwertiger Alkohole und deren ethoxylierte Derivate (z. B. Glycerylmonostearat, Sorbitanstearat, Glycerylstearylcitrat, Sucrosestearat)
b) ethoxylierte Fettalkohole und Fettsäuren
c) ethoxylierte Fettamine, Fettsäureamide, Fettsäurealkanolamide
d) Alkylphenolpolyglycolether (z.B. Triton X)

Erfindungsgemäß bevorzugter Emulgator ist das Glycerylstearatcitrat. Dieser ist beispielsweise erhältlich unter der Produktbezeichnung "IMWITOR® 370" der Gesellschaft Hüls AG.

Bevorzugt sind Zubereitungen, welche dadurch gekennzeichnet sind, daß sie zusätzlich enthalten
(vi) einen oder mehrere Fettalkohole,
   - wobei der Gesamtgehalt der Fettalkohole vorteilhaft gewählt wird aus dem Konzentrationsbereich von 0 bis 10 Gew.-%, vorzugsweise aus dem Bereich von 0 bis 5,0 Gew.-%, besonders bevorzugt 0 bis 3,0 Gew.-%, jeweils bezogen auf das Gesamtgewicht der Zubereitungen.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Es ist auch von Vorteil, den erfindungsgemäßen Zubereitungen Antioxidantien zuzusetzen. Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Car nosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können öllösliche Antioxidantien eingesetzt werden.

Eine erstaunliche Eigenschaft der vorliegenden Erfindung ist, daß erfindungsgemäße Zubereitungen sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Bevorzugte Antioxidantien sind dabei Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist dem Fachmann natürlich bekannt, daß kosmetische Zubereitungen zumeist nicht ohne die üblichen Hilfs- und Zusatzstoffe denkbar sind. Die erfindungsgemäßen kosmetischen und dermatologischen Zubereitungen können dementsprechend ferner kosmetische Hilfsstoffe enthalten, wie sie üblicherweise in solchen Zubereitungen verwendet werden, beispielsweise Konsistenzgeber, Stabilisatoren, Füllstoffe, Konservierungsmittel, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, entzündungshemmende Substanzen, zusätzliche Wirkstoffe wie Vitamine oder Proteine, Lichtschutzmittel, Insektenrepellentien, Bakterizide, Viruzide, Wasser, Salze, antimikrobiell, proteolytisch oder keratolytisch wirksame Substanzen, Medikamente oder andere übliche Bestandteile einer kosmetischen oder dermatologischen Formulierung wie Alkohole, Polyole, Polymere, Schaumstabilisatoren, organische Lösungsmittel oder auch Elektrolyte.

Letztere können beispielsweise, gewählt werden aus der Gruppe der Salze mit folgenden Anionen: Chloride, ferner anorganische Oxo-Element-Anionen, von diesen insbesondere Sulfate, Carbonate, Phosphate, Borate und Aluminate. Auch auf organischen Anionen basierende Elektrolyte sind vorteilhaft, z.B. Lactate, Acetate, Benzoate, Propionate, Tartrate, Citrate, Aminosäuren, Ethylendiamintetraessigsäure und deren Salze und andere mehr. Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Besonders bevorzugt sind Kaliumchlorid, Kochsalz, Magnesiumsulfat, Zinksulfat und Mischungen daraus.

Mutatis mutandis gelten entsprechende Anforderungen an die Formulierung medizinischer Zubereitungen.

Die erfindungsgemäßen Gelcrèmes können als Grundlage für kosmetische oder dermatologische Formulierungen dienen. Diese können wie üblich zusammengesetzt sein und beispielsweise zur Behandlung und der Pflege der Haut und/oder der Haare, als Lippenpflegeprodukt, als Deoprodukt und als Schmink- bzw. Abschminkprodukt in der dekorativen Kosmetik oder als Lichtschutzpräparat dienen. Zur Anwendung werden die erfindungsgemäßen kosmetischen und dermatologischen Zubereitungen in der für Kosmetika oder Dermatika üblichen Weise auf die Haut und/oder die Haare in ausreichender Menge aufgebracht.

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcreme, Tages- oder Nachtcreme usw. Es ist gegebenenfalls möglich und vorteilhaft, die erfindungsgemäßen Zusammensetzungen als Grundlage für pharmazeutische Formulierungen zu verwenden.

Die kosmetischen oder dermatologischen Mittel gemäß der Erfindung können beispielsweise als aus Aerosolbehältern, Quetschflaschen oder durch eine Pumpvorrichtung versprühbare Präparate vorliegen oder in Form einer mittels Roll-on-Vorrichtungen auftragbaren flüssigen Zusammensetzung, jedoch auch in Form einer aus normalen Flaschen und Behältern auftragbaren Emulsion.

Als Treibmittel für aus Aerosolbehältern versprühbare kosmetische oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffkombinationen zusätzlich mindestens eine UV-A-Filtersubstanz und/oder mindestens eine UV-B-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z.B. in Tagescremes gewöhnlich UV-A- bzw. UV-B-Filtersubstanzen eingearbeitet.

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die erfindungsgemäßen Emulsionen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester,
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- Derivate des 1,3,5-Triazins, vorzugsweise 2,4,6-Trianilino-(p-carbo-2'-ethyl-1'-hexyloxy) -1,3,5-triazin.

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, erfindungsgemäße Lipodispersionen mit UVA-Filtem zu formulieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion.

Erfindungsgemäße kosmetische und dermatologische Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Als weitere Bestandteile können verwendet werden:
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.

Die nachfolgenden Beispiele soll die vorliegende Erfindung verdeutlichen, ohne sie einzuschränken.

### Beispiele

### (1) Gelcrème

| | Gew.-% |
|---|---|
| Glycerylstearatcitrat | 1,00 |
| Hydrierte Kokosfettsäure Glyceride | 1,00 |
| Dicaprylylcarbonat | 3,00 |
| Hydroxyethylcellulose | 0,375 |
| Xanthan Gummi | 0,125 |
| Carbomer | 0,125 |
| Dimethicon | 3,00 |
| NaOH | 0,31 |
| Hydriertes Polyisobuten | 3,00 |
| Glycerin | 5,00 |
| Parfum | q.s. |
| Konservierungsmittel | q.s. |
| Wasser | 100,00 |

### (2) Gellotion

| | Gew.-% |
|---|---|
| Glycerylstearatcitrat | 1,00 |
| Hydrierte Kokosfettsäure Glyceride | 0,50 |
| Dicaprylylcarbonat | 1,00 |
| Hydroxyethylcellulose | 0,25 |
| Carbomer | 0,15 |
| Cyclomethicon | 5,00 |
| NaOH | 0,10 |
| Hydriertes Polyisobuten | 5,00 |
| Glycerin | 12,00 |
| Parfum | q.s. |
| Konservierungsmittel | q.s. |
| Wasser | 100,00 |

## Patentansprüche

1. Kosmetische Zubereitungen in Form von Gelemulsionen auf Zubereitungen des Typs O/W-Emulsion mit einem Gehalt von
- Hydrokolloiden, gewählt aus der Gruppe
(i) Hydroxyethylcellulose
(ii) Xanthan Gummi
(iii) Carbomer
basierend
- wobei das Verhältnis aus (i) : (ii) : (iii) wie a : b : c gewählt wird, wobei a, b und c unabhängig voneinander rationale Zahlen von 1 bis 5, wobei b aber auch den Wert Null annehmen kann,
- wobei (i), (ii) und (iii) in den Zubereitungen in Gesamtkonzentrationen (a + b + c) von 0,25 - 1,5 Gew.-% vorliegen, bezogen auf das Gesamtgewicht der Zubereitungen,
- wobei diese Zubereitungen ferner enthalten
(iv) ein oder mehrere Lipide,
- wobei der Gesamtgehalt der Lipide gewählt wird aus dem Konzentrationsbereich von 3,0 bis 20,0 Gew.-%, bezogen auf das Gesamtgewicht der Zubereitungen,
- wobei diese Zubereitungen ferner enthalten
(v) einen oder mehrere nichtionische und/oder anionische Emulgatoren mit HLB-Werten zwischen 8 und 16,
- wobei der Gesamtgehalt der Emulgatoren 1,5 Gew.-% nicht übersteigt.
mit Ausnahme solcher Zubereitungen, bestehend aus
| | Gew.-% |
|---|---|
| Trilaureth-4 Phosphat | 0,4 |
| Dimethicon | 2 |
| Caprylic/Caprictriglycerid | 5 |
| C₁₂₋₁₅ Alkylbenzoat | 5 |
| Vitamen E-Acetat | 0,5 |
| Anisotriazin | 2 |
| Octylmethoxycinnamat | 8 |
| Titandioxid | 1,5 |
| Konservierung | 0,5 |
| Glycerin | 6 |
| Xanthan Gummi | 0,1 |
| Carbopol 981® | 0,5 |
| Bisimidazylat | 2 |
| Wasser | ad 100 |
oder
| | Gew.-% |
|---|---|
| Isostearinsäure | 0,2 |
| Trilaureth-4 Phosphat | 0,25 |
| Caprylic/Caprictriglycerid | 5 |
| Dicaprylylether | 5 |
| Butylenglykoldicaprylat/Caprat | 2 |
| Vitamen E-Acetat | 0,5 |
| Titandioxid | 3 |
| Konservierung | 0,5 |
| Glycerin | 10 |
| Xanthan Gummi | 0,2 |
| Carbopol 981® | 0,2 |
| Bisimidazylat | 2 |
| Wasser | ad 100 |

2. Zubereitungen nach Anspruch 1,
- wobei das Verhältnis aus (i) : (ii) : (iii) wie a : b : c gewählt wird, wobei a, b und c unabhängig voneinander rationale Zahlen von 1 bis 3 darstellen:

3. Zubereitungen nach Anspruch 1,
- wobei (i), (ii) und (iii) in den Zubereitungen in Gesamtkonzentrationen (a + b + c) von 0,5 - 1,0 Gew.-% vorliegen, bezogen auf das Gesamtgewicht der Zubereitungen.

4. Zubereitungen nach Anspruch 1,
(iv) wobei der Gesamtgehalt der Lipide gewählt wird aus dem Konzentrationsbereich von 7,5 bis 15 Gew.-%.

5. Zubereitungen nach Anspruch 1, enthaltend
(v) einen oder mehrere nichtionische und/oder anionische Emulgatoren mit HLB-Werten zwischen 10 und 12.

6. Zubereitungen nach Anspruch 1,
(v) wobei der Gesamtgehalt der Emulgatoren aus dem Konzentrationsbereich von 0,5 bis 1,0 Gew.-% gewählt wird, bezogen auf das Gesamtgewicht der Zubereitungen.

7. Zubereitungen nach Anspruch 1, **dadurch gekennzeichnet, daß**
(v) als Emulgator Glycerylstearatcitrat gewählt wird.

8. Zubereitungen nach Anspruch 1, **dadurch gekennzeichnet, daß** sie zusätzlich enthalten
(vi) einen oder mehrere Fettalkohole.

9. Zubereitungen nach Anspruch 8, **dadurch gekennzeichnet, daß**
(vi) der Gesamtgehalt der Fettalkohole gewählt wird aus dem Konzentrationsbereich von 0 bis 10 Gew.-%, vorzugsweise aus dem Bereich von 0 bis 5,0 Gew.-%, besonders bevorzugt 0 bis 3,0 Gew.-%, jeweils bezogen auf das Gesamtgewicht der Zubereitungen.

## Claims

1. Cosmetic preparations in the form of gel emulsions based on preparations of the O/W emulsion type with a content of
- hydrocolloids chosen from the group
i) hydroxyethylcellulose
ii) xanthan gum
iii) carbomer,
- where the ratio of (i) : (ii) : (iii) is chosen as a : b : c, where a, b and c, independently of one another, can assume rational numbers from 1 to 5, where b can also assume the value 0,
- where (i), (ii) and (iii) are present in the preparations in total concentrations (a + b + c) of 0.25-1.5% by weight, based on the total weight of the preparations,
- where these preparations further comprise
(iv) one or more lipids,
- where the total content of the lipids is chosen from the concentration range from 3.0 to 20.0% by weight, based on the total weight of the preparations,
- where these preparations further comprise
(v) one or more nonionic and/or anionic emulsifiers with HLB values between 8 and 16,
- where the total content of the emulsifiers does not exceed 1.5% by weight with the exception of those preparations consisting of
| | % by weight |
|---|---|
| Trilaeueth-4 phosphate | 0.4 |
| Dimethicone | 2 |
| Caprylic/capric triglyceride | 5 |
| C₁₂₋₁₅ alkyl benzoate | 5 |
| Vitamin E acetate | 0.5 |
| Anisotriazine | 2 |
| Octyl methoxycinnamate | 8 |
| Titanium dioxide | 1.5 |
| Preservative | 0.5 |
| Glycerol | 6 |
| Xanthan gum | 0.1 |
| Carbopol 981® | 0.5 |
| Bisimidazylate | 2 |
| Water | ad 100 |
or
| | % by weight |
|---|---|
| Isostearic acid | 0.2 |
| Trilaureth-4 phosphate | 0.25 |
| Caprylic/capric triglyceride | 5 |
| Dicaprylyl ether | 5 |
| Butylene glycol dicaprylate/caprate | 2 |
| Vitamin E acetate | 0.5 |
| Titanium dioxide | 3 |
| Preservative | 0.5 |
| Glycerol | 10 |
| Xanthan gum | 0.2 |
| Carbopol 981® | 0.2 |
| Bisimidazylate | 2 |
| Water | ad 100 |

2. Preparations according to Claim 1,
- where the ratio of (i) : (ii) : (iii) is chosen as a : b : c, where a, b and c, independently of one another, represent rational numbers from 1 to 3.

3. Preparations according to Claim 1,
- where (i), (ii) and (iii) are present in the preparations in total concentrations (a + b + c) of 0.5-1.0% by weight, based on the total weight of the preparations.

4. Preparations according to Claim 1,
(iv) where the total content of the lipids is chosen from the concentration range from 7.5 to 15% by weight.

5. Preparations according to Claim 1, comprising
(v) one or more nonionic and/or anionic emulsifiers with HLB values between 10 and 12.

6. Preparations according to Claim 1,
(v) where the total content of the emulsifiers is chosen from the concentration range from 0.5 to 1.0% by weight, based on the total weight of the preparations.

7. Preparations according to Claim 1, **characterized in that**
(v) glyceryl stearate citrate is chosen as emulsifier.

8. Preparations according to Claim 1, **characterized in that** they additionally comprise
(vi) one or more fatty alcohols.

9. Preparations according to Claim 8, **characterized in that**
(vi) the total content of the fatty alcohols is chosen from the concentration range from 0 to 10% by weight, preferably from the range from 0 to 5.0% by weight, particularly preferably 0 to 3.0% by weight, in each case based on the total weight of the preparations.

## Revendications

1. Préparations cosmétiques sous la forme d'émulsions de gel telles que des préparations du type émulsion H/E ayant une teneur
- en hydro colloïdes, sélectionnés dans le groupe constitué
(i) de l'hydroxyéthylcellulose
(ii) de la gomme de xanthane
(iii) des carbomères
- le rapport (i) : (ii) : (iii) étant sélectionné comme le rapport a : b : c, a, b et c étant indépendamment les uns des autres des nombres rationnels allant de 1 à 5, b pouvant cependant prendre la valeur zéro,
(i), (ii) et (iii) étant présents dans les préparations dans des concentrations globales (a + b + c) de 0,25 - 1,5 % en poids, par rapport au poids total des préparations,
- ces préparations contenant en outre
(iv) un ou plusieurs lipides,
- la teneur globale en lipides étant sélectionnée dans le domaine des concentrations allant de 3,0 à 20,0 % en poids, par rapport au poids total des préparations,
- ces préparations contenant en outre
(v) un ou plusieurs émulsifiants non ioniques et/ou anioniques ayant des valeurs HLB comprises entre 8 et 16, la teneur globale des émulsifiants ne dépassant pas 1,5 % en poids,
à l'exception de préparations de ce genre se composant de
| | % en poids |
|---|---|
| Phosphate de trilaureth-4 | 0,4 |
| Diméthicone | 2 |
| Triglycéride caprylique/caprique | 5 |
| Benzoate de C_{12 15}-alkyle | 5 |
| Acétate de la vitamine E | 0,5 |
| Anisotriazine | 2 |
| Méthoxycinnamate d'octyle | 8 |
| Dioxyde de titane | 1,5 |
| | |
|---|---|
| Agent de conservation | 0,5 |
| Glycérine | 6 |
| Gomme de xanthane | 0,1 |
| Produit Carbopol 981 ® | 0,5 |
| Bisimidazylate | 2 |
| Eau | à 100 % |
ou
| | % en poids |
|---|---|
| Acide isostéarinique | 0,2 |
| Phosphate de trilaureth-4 | 0,25 |
| Triglycéride caprylique/caprique | 5 |
| Ether dicaprylylique | 5 |
| Dicaprylate/caprate de butylèneglycol | 2 |
| Acétate de la vitamine E | 0,5 |
| Dioxyde de titane | 3 |
| Agent de conservation | 0,5 |
| Glycérine | 10 |
| Gomme de xanthane | 0,2 |
| Produit Carbopol 981 ® | 0,2 |
| Bisimidazylate | 2 |
| Eau | à 100 |

2. Préparations selon la revendication 1, le rapport de (i) : (ii) : (iii) étant sélectionné comme le rapport de a : b : c, a, b et c représentant, indépendamment les uns des autres, des nombres rationnels de 1 à 3.

3. Préparations selon la revendication 1,
(i), (ii) et (iii) étant présents dans les préparations dans des concentrations globales (a + b + c) de 0,5 - 1,0 % en poids, par rapport au poids total des préparations.

4. Préparations selon la revendication 1, (iv) la teneur globale en lipides étant sélectionnée dans le domaine de concentration allant de 7,5 à 15 % en poids.

5. Préparations selon la revendication 1,
un ou plusieurs émulsifiants non ioniques et/ou anioniques ayant des valeurs HLB comprises entre 10 et 12.

6. Préparations selon la revendication 1,
(vi) la teneur globale en émulsifiants étant sélectionnée dans le domaine de concentration allant de 0,5 à 1,0 % en poids, par rapport au poids total des préparations.

7. Préparations selon la revendication 1, **caractérisées** (v) en ce que l'on sélectionne, en tant qu'émulsifiant, le stéarate/citrate de glycéryle.

8. Préparations selon la revendication 1, **caractérisées en ce qu'**elles contiennent en sus
(vi) un ou plusieurs alcools gras.

9. Préparations selon la revendication 8, **caractérisées en ce que**
(vi) la teneur globale en alcools gras est sélectionnée dans le domaine de concentration allant de 0 à 10 % en poids, de préférence, de 0 à 5,0 % en poids, en particulier, de préférence, de 0 à 3,0 % en poids, par rapport à chaque fois au poids total des préparations.
